# EUROPEAN PATENT APPLICATION

(11) **EP 2 902 479 A1**
(43) Date of publication of application: **05.08.2015**
(21) Application number: 13842015.3
(22) Date of filing: 12.08.2013
(51) Int. Cl.: C12N 1/00, C12Q 1/04, C12Q 1/68

(54) **CULTURE MEDIUM FOR FOOD POISONING BACTERIA, AND METHOD FOR DETECTING FOOD POISONING BACTERIA**

(30) Priority: 26.09.2012 JP 2012212231
(71) Applicant: Toyo Seikan Group Holdings, Ltd., Shinagawa-ku Tokyo 141-8627 (JP)
(72) Inventor: YAMASAKI, Takaaki, Yokohama-shi Kanagawa 240-0062 (JP); MIYANO, Motohiro, Yokohama-shi Kanagawa 230-0001 (JP); NAKAJIMA, Kazuki, Yokohama-shi Kanagawa 240-0062 (JP); YOSHIDA, Mitsuhiro, Yokohama-shi Kanagawa 240-0062 (JP)
(74) Representative: Pestalozzi, Deborah
(86) International application number: PCT/JP2013/004834
(87) International publication number: WO 2014/049938

(57) **Abstract**

A culture medium for food poisoning bacteria makes it possible to appropriately allow a plurality of types of food poisoning bacteria including *Staphylococcus aureus* to grow at the same time in a single culture medium. The culture medium for food poisoning bacteria includes a peptone, a yeast extract, and magnesium sulfate. A method for detecting food poisoning bacteria includes allowing a plurality of types of food poisoning bacteria including at least *Staphylococcus aureus* to grow at the same time using a culture medium that includes a peptone, a yeast extract, and magnesium sulfate, extracting DNA from the plurality of types of food poisoning bacteria that have been allowed to grow, adding a primer for specifically amplifying an amplification target region of the DNA of each of the plurality of types of food poisoning bacteria to a PCR solution, amplifying the amplification target region by PCR, and detecting the resulting amplified product.

## Description

### TECHNICAL FIELD

The invention relates to technology that incubates food poisoning bacteria. In particular, the invention relates to technology that incubates a plurality of types of food poisoning bacteria at the same time in a single culture medium.

### BACKGROUND ART

In recent years, more than one thousand food poisoning cases occur each year. When a food poisoning case has occurred, the detection target bacteria are grown, and the type thereof is determined in order to specify the causative food poisoning bacteria. When growing the detection target bacteria, all of the detection target bacteria that are considered to be causative of food poisoning based on the experience of public health engineers are independently incubated. In this case, it is necessary to change the culture medium, the culture conditions, and the like corresponding to each type of detection target bacteria.

This approach has a problem in that it takes a lot of time and money to incubate and detect the food poisoning bacteria.

Therefore, research and development of technology that incubates a plurality of types of food poisoning bacteria at the same time in a single culture medium have been undertaken in order to easily and promptly culture and detect food poisoning bacteria, and reduce the amount of waste (expendable supplies).

For example, Patent Document 1 states that six types of food poisoning bacteria including *Staphylococcus aureus, Salmonella, Escherichia coli, Vibrio, Bacillus cereus,* and *Listeria* can be incubated at the same time using the method disclosed in Patent Document 1.

Patent Document 2 states that a plurality of types of food poisoning bacteria including *Listeria* that cannot be easily grown, can be incubated at the same time using the method disclosed in Patent Document 2.

Patent Document 1: JP-A-2008-48670
Patent Document 2: Japanese Patent No. 4621919

### SUMMARY OF THE INVENTION

### TECHNICAL PROBLEM

However, the culture medium used in Patent Document 1 has a problem in that it is difficult to culture the detection target bacteria in a stable manner. In particular, when detection target bacteria having a high doubling rate, or non-detection target bacteria such as contaminating bacteria, are included in the culture medium, the doubling rate of some of the detection target bacteria decreases due to the effects of such bacteria, and the detection target bacteria may not appropriately grow.

The culture medium used in Patent Document 2 has a problem in that *Staphylococcus aureus* does not sufficiently grow, and cannot be appropriately detected.

Since Patent Documents 1 and 2 aim to allow the detection target bacteria to grow to a concentration of 10³ cfu/ml or more, sensitivity is insufficient when applying the methods (culture media) disclosed in Patent Documents 1 and 2 to a rapid detection method or the like (i.e., the methods (culture media) disclosed in Patent Documents 1 and 2 lack versatility).

The inventors of the invention conducted extensive studies, and succeeded in developing a culture medium that makes it possible to allow four types of food poisoning bacteria (i.e., *Escherichia coli, Salmonella* sp., *Staphylococcus aureus,* and *Vibrio parahaemolyticus)* that are important for food inspection, epidemiologic environmental inspection, environmental inspection, clinical examination, and livestock health management, to grow at the same time.

Since the detection lower limit of a normal rapid detection method (e.g., immunochromatographic assay or ELISA) is 10⁵ cfu/ml, it is desirable to allow the detection target bacteria to grow to a concentration of 10⁵ cfu/ml or more within a practical culture time taking account of a situation in which the culture medium is used for such a detection method. It is necessary to analyze food poisoning bacteria with regard to toxin production by performing a biochemical assay. In the previous food poisoning cases due to *Escherichia coli, Salmonella* sp., *Staphylococcus aureus,* and *Vibrio parahaemolyticus,* a toxin was produced (i.e., food poisoning occurred) when the detection target bacteria were present at a concentration of 10⁵ cfu/ml or more. Therefore, it is desirable to allow the detection target bacteria to grow to a concentration of 10⁵ cfu/ml or more from this point of view.

When assaying a plurality of types of food poisoning bacteria at the same time, it is important to take account of the difference in bacterial count between different types of food poisoning bacteria. Specifically, when using a rapid detection method that utilizes multiplex PCR or the like, it is difficult to appropriately amplify (and detect) food poisoning bacteria present at a relatively low concentration when amplifying a plurality of types of food poisoning bacteria at the same time if the difference in bacterial count is 10³ cfu/ml or more. Therefore, it is desirable to amplify a plurality of types of food poisoning bacteria so that the difference in bacterial count is less than 10³ cfu/ml.

The invention was conceived in view of the above situation. An object of the invention is to provide a culture medium for food poisoning bacteria that makes it possible to appropriately allow a plurality of types of food poisoning bacteria including *Staphylococcus aureus* to grow at the same time, and a method for detecting food poisoning bacteria.

### SOLUTION TO PROBLEM

A culture medium for food poisoning bacteria according to the invention that achieves the above object includes a peptone, a yeast extract, and magnesium sulfate.

A method for detecting food poisoning bacteria according to the invention includes allowing a plurality of types of food poisoning bacteria including at least *Staphylococcus aureus* to grow at the same time using a culture medium that includes a peptone, a yeast extract, and magnesium sulfate, extracting DNAfrom the plurality of types of food poisoning bacteria that have been allowed to grow, adding primers for specifically amplifying an amplification target region of the DNA of each of the plurality of types of food poisoning bacteria to a PCR solution, amplifying the amplification target region by PCR, and detecting the resulting amplified product.

The invention thus makes it possible to appropriately allow a plurality of types of food poisoning bacteria including *Staphylococcus aureus* to grow at the same time in a single culture medium.

### BRIEF DESCRIPTION OF DRAWINGS

FIG. 1 is a view showing the results of Experiment 1 (verification regarding the culture medium according to the embodiments of the invention that includes a peptone, a yeast extract, and magnesium sulfate).
FIG. 2 is a view showing the results of Experiment 2 (verification regarding the optimum peptone concentration in the culture medium according to the embodiments of the invention).
FIG. 3 is a view showing the results of Experiment 3 (verification regarding the optimum sodium chloride concentration in the culture medium according to the embodiments of the invention).
FIG. 4 is a view showing the results of Experiment 4 (verification regarding the optimum glucose concentration in the culture medium according to the embodiments of the invention).
FIG. 5 is a view showing the results of Experiment 5 (verification regarding the optimum pH of the culture medium according to the embodiments of the invention).
FIG. 6 is a view showing the results of Experiment 6 (verification regarding the bacterial count after completion of culture using the culture medium according to the embodiments of the invention and an existing culture medium).
FIG. 7 is a view showing the results of Experiment 7 (verification regarding the culture medium according to the embodiments of the invention when contaminating bacteria are mixed).
FIG. 8 is a view showing the results of Experiment 8 (verification regarding the culture medium according to the embodiments of the invention when food is added).
FIG. 9 is a view showing the base sequence of each primer for specifically amplifying the amplification target region of the DNA of food poisoning bacteria used in each experiment.
FIG. 10 is a view showing the electrophoretic detection results for specifically amplified products obtained by multiplex PCR using the DNA of food poisoning bacteria incubated in Experiment 1.
FIG. 11 is a view showing the electrophoretic detection results (sodium chloride concentration: 0 to 1.5%) for specifically amplified products obtained by multiplex PCR using the DNA of food poisoning bacteria incubated in Experiment 3.
FIG. 12 is a view showing the electrophoretic detection results (sodium chloride concentration: 2.0 to 3.5%) for specifically amplified products obtained by multiplex PCR using the DNA of food poisoning bacteria incubated in Experiment 3.

### DESCRIPTION OF EMBODIMENTS

A culture medium for food poisoning bacteria, and a method for detecting food poisoning bacteria according to the embodiments of the invention are described in detail below.

A culture medium for food poisoning bacteria according to the embodiments of the invention includes a peptone, a yeast extract, and magnesium sulfate. The culture medium for food poisoning bacteria according to the embodiments of the invention that has the above configuration makes it possible to allow a plurality of types of food poisoning bacteria including at least *Staphylococcus aureus* to grow at the same time. In particular, it is possible to appropriately allow *Escherichia coli, Salmonella* sp., *Staphylococcus aureus,* and *Vibrio parahaemolyticus* to grow at the same time.

### Detection target bacteria

### Escherichia coli

*Escherichia coli* is a gram-negative facultative anaerobic bacillus. *Escherichia coli* is one type of intestinal bacteria, and most of them are harmless. However, some of them cause stomachache, diarrhea, or the like, and are referred to as "pathogenic *Escherichia coli".* The growth pH (optimum pH) for enterohemorrhagic *E. coli* (EHEC) (e.g., 0157) is 4.4 to 9.0 (6.0 to 7.6), and the growth temperature (optimum temperature) is 7.0 to 46.0 (35 to 40) °C. An mEC medium is normally used as the culture medium for *Escherichia coli.*

### Salmonella

*Salmonella* sp. is a gram-negative facultative anaerobic bacillus, and is one type of intestinal bacteria. Some of them cause infectious food poisoning. The growth pH (optimum pH) for *Salmonella* sp. is 3.8 to 9.5 (7.0 to 7.5), and the growth temperature (optimum temperature) is 5.2 to 46.2 (35 to 43) °C. Buffered peptone water is normally used as the culture medium for *Salmonella* sp.

### Staphylococcus aureus

*Staphylococcus aureus* is one type of *Staphylococcus,* and is a gram-positive facultative anaerobic micrococcus. *Staphylococcus aureus* resides on human skin and in the nasal cavity and intestines, and may cause diseases even in healthy people. However, the toxicity of *Staphylococcus aureus* is normally weak when the number thereof is small. *Staphylococcus aureus* may cause food poisoning, and various infectious diseases such as epidermal infections, pneumonia, and meningitis. The growth pH (optimum pH) for *Staphylococcus aureus* is 4.2 to 9.3 (7.0 to 7.5), and the growth temperature (optimum temperature) is 6.5 to 46.0 (30 to 37) °C. A salt-containing tryptic soy broth medium is normally used as the culture medium for *Staphylococcus aureus.*

### Vibrio parahaemolyticus

*Vibrio parahaemolyticus* is a gram-negative halophilic bacillus. *Vibrio parahaemolyticus* is mainly found in seawater, and causes a human to develop food poisoning through infection. The growth pH (optimum pH) for *Vibrio parahaemolyticus* is 5.5 to 9.6 (7.6 to 8.0), and the growth temperature (optimum temperature) is 10.0 to 43.0 (35 to 37) °C. Alkaline peptone water is normally used as the culture medium for *Vibrio parahaemolyticus.*

Only *Staphylococcus aureus* among these food poisoning bacteria is gram-positive. Since the growth rate of gram-positive bacteria is lower than that of gram-negative bacteria, it has been very difficult to culture these bacteria at the same time in a single (identical) culture medium.

Specifically, when these food poisoning bacteria are simultaneously incubated in a single culture medium, *Escherichia coli* and *Salmonella* sp. grow rapidly, and the growth of *Staphylococcus aureus* and *Vibrio parahaemolyticus* is thereby suppressed. In particular, it has been difficult to allow *Staphylococcus aureus* to sufficiently grow. Sodium chloride is indispensable for the growth of *Vibrio parahaemolyticus.* However, the sodium chloride concentration in the culture medium affects the growth of other bacteria.

Therefore, the culture medium for food poisoning bacteria according to the embodiments of the invention is designed to have a composition that makes it possible to allow *Escherichia coli, Salmonella* sp., *Staphylococcus aureus,* and *Vibrio parahaemolyticus* to grow in a well-balanced manner.

### Composition of culture medium

### Peptone

A peptone is obtained by hydrolyzing animal or plant proteins into amino acids and low-molecular-weight peptides. In the examples described below, Bacto Tryptone manufactured by Nippon Becton Dickinson Company, Ltd. was used as the peptone.

It is preferable to adjust the peptone concentration in the culture medium to 1% w/v or more (mass/volume percentage). If the peptone concentration is less than 1% w/v, it may be difficult to allow *Vibrio parahaemolyticus* to sufficiently grow. It is possible to satisfactorily allow *Escherichia coli, Salmonella* sp., *Staphylococcus aureus,* and *Vibrio parahaemolyticus* to sufficiently grow even when the peptone concentration is 5% w/v or more. Therefore, it is preferable to adjust the peptone concentration in the culture medium to 1% w/v to 5% w/v, for example.

### Yeast extract

The term "yeast extract" used herein refers to an extract that is extracted from yeast through self-digestion or treatment with an enzyme, hot water, or the like, and includes amino acids, nucleic acids, minerals, vitamins, and the like. In the examples described below, Bacto Yeast Extract manufactured by Nippon Becton Dickinson Company, Ltd., was used as the yeast extract.

It is preferable that the culture medium include the yeast extract even in a small amount. It is preferable that the culture medium include the yeast extract at a concentration of less than 0.6% w/v. Since the yeast extract includes an insoluble component, precipitation may occur after treatment using an autoclave if the yeast extract concentration in the culture medium is higher than 0.6% w/v, and the detection target bacteria may not grow to a concentration of 10⁵ cfu/ml or more, or the growth of the detection target bacteria may become unstable. It is possible to satisfactorily allow *Escherichia coli, Salmonella* sp., *Staphylococcus aureus,* and *Vibrio parahaemolyticus* to grow at the same time in a single culture medium by adjusting the yeast extract concentration in the culture medium to 0.0001% w/v to 0.6% w/v, for example.

### Magnesium sulfate

Magnesium sulfate is a salt of sulfuric acid and magnesium. In the embodiments of the invention, magnesium sulfate heptahydrate is used as magnesium sulfate. In the examples described below, magnesium sulfate heptahydrate manufactured by Wako Pure Chemical Industries, Ltd. was used as magnesium sulfate.

It is preferable that the culture medium include magnesium sulfate even in a small amount. It is preferable that the culture medium include magnesium sulfate at a concentration of less than 0.1 % w/v. If the magnesium sulfate concentration in the culture medium is too high, the growth of the food poisoning bacteria may be adversely affected. Magnesium sulfate reacts with a phosphate, and precipitates. Therefore, precipitation may occur after treatment using an autoclave if the magnesium sulfate concentration in the culture medium is larger than 0.1 % w/v, and the detection target bacteria may not grow to a concentration of 10⁵ cfu/ml or more, or the growth of the detection target bacteria may become unstable. It is possible to satisfactorily allow *Escherichia coli, Salmonella* sp., *Staphylococcus aureus,* and *Vibrio parahaemolyticus* to grow at the same time in a single culture medium by adjusting the magnesium sulfate concentration in the culture medium to 0.0001% w/v to 0.1 % w/v, for example.

### Sodium chloride

Sodium chloride (NaCl) is indispensable for the growth of *Vibrio parahaemolyticus.* In the examples described below, sodium chloride manufactured by Wako Pure Chemical Industries, Ltd. was used as sodium chloride.

It is preferable to adjust the sodium chloride concentration in the culture medium to 0.5% w/v to 3.0% w/v. If the sodium chloride concentration is less than 0.5% w/v, *Vibrio parahaemolyticus* may not grow. If the sodium chloride concentration exceeds 3.0% w/v, the growth of *Escherichia coli* and *Salmonella* sp. may be affected. In particular, it is possible to satisfactorily allow *Staphylococcus aureus* and *Vibrio parahaemolyticus* to grow when the sodium chloride concentration is 1.5% w/v to 3.0% w/v. It is possible to allow *Escherichia coli, Salmonella* sp., *Staphylococcus aureus,* and *Vibrio parahaemolyticus* to grow in the most well-balanced manner when the sodium chloride concentration is 1.5% w/v to 2.0% w/v.

### Additional component

The culture medium according to the embodiments of the invention may include a phosphate (0.35% w/v disodium hydrogen phosphate + 0.15% w/v potassium dihydrogen phosphate) as an additional component. The phosphate concentration in the culture medium is not particularly limited as long as it is possible to satisfactorily allow *Escherichia coli, Salmonella* sp., *Staphylococcus aureus,* and *Vibrio parahaemolyticus* to grow. The content ratio of disodium hydrogen phosphate and potassium dihydrogen phosphate is not particularly limited as long as it is possible to satisfactorily allow *Escherichia coli, Salmonella* sp., *Staphylococcus aureus,* and *Vibrio parahaemolyticus* to grow.

The culture medium according to the embodiments of the invention may include a further component as long as the culture medium includes the peptone, the yeast extract, and magnesium sulfate.

### pH of culture medium

It is preferable to adjust the pH of the culture medium according to the embodiments of the invention to 6.5 to 7.5. When the pH of the culture medium is within the above range, it is possible to allow *Escherichia coli, Salmonella* sp., *Staphylococcus aureus,* and *Vibrio parahaemolyticus* to grow at the same time. If the pH of the culture medium is lower than or higher than the above range, the growth of *Staphylococcus aureus* may be insufficient.

### Method for producing culture medium

The culture medium according to the embodiments of the invention may be produced by mixing the peptone, the yeast extract, magnesium sulfate, sodium chloride, disodium hydrogen phosphate, and potassium dihydrogen phosphate, adjusting the pH of the mixture to 7.0, and sterilizing the mixture at 121°C for 15 minutes using an autoclave.

### Method for using culture medium

The culture medium according to the embodiments of the invention may be used as described below, for example.

The culture medium according to the embodiments of the invention may be used to allow *Escherichia coli, Salmonella* sp., *Staphylococcus aureus,* and *Vibrio parahaemolyticus* included in a sample to grow to the detectable level. Examples of the sample include food, a clinical sample (feces or vomit), and the like.

For example, when using food as the sample, the presence or absence of food poisoning bacteria can be determined by adding 25 g of the food sample to 225 mL of the culture medium, culturing *Escherichia coli, Salmonella* sp., *Staphylococcus aureus,* and *Vibrio parahaemolyticus* at 37°C for 20 hours, selectively detecting the respective food poisoning bacteria. In this case, the number of each type of food poisoning bacteria thus incubated may be counted, and a qualitative determination may be performed, for example.

The number of each type of food poisoning bacteria may be counted by adding a serial diluent of the culture to a selective medium corresponding to each type of food poisoning bacteria, culturing the bacteria for a specific time, and measuring the typical colony count of each type of bacteria. The qualitative determination may be performed by selecting bacteria using a culture medium or an immunological selection method.

### Method for detecting food poisoning bacteria

The method for detecting food poisoning bacteria according to the embodiments of the invention may be implemented as described below using the culture medium according to the embodiments of the invention.

Specifically, a plurality of types of food poisoning bacteria including at least *Staphylococcus aureus* are allowed to grow at the same time using the culture medium that includes the peptone, the yeast extract, and magnesium sulfate, and DNA is extracted from the plurality of types of food poisoning bacteria thus allowed to grow. Primers for specifically amplifying the amplification target region of the DNA of each of the plurality of types of food poisoning bacteria are added to a PCR solution. The amplification target region is amplified by PCR, and the resulting amplified product is detected.

More specifically, the culture medium (culture) in which the food poisoning bacteria have been incubated is collected, and centrifuged. After removing the supernatant liquid, a lysozyme aqueous solution suitable for bacteriolysis of gram-positive bacteria is added to the precipitate, and a bacteriolysis treatment is performed. Proteolysis and column purification are performed to obtain a DNA extract, which is used as a sample to be amplified by multiplex PCR.

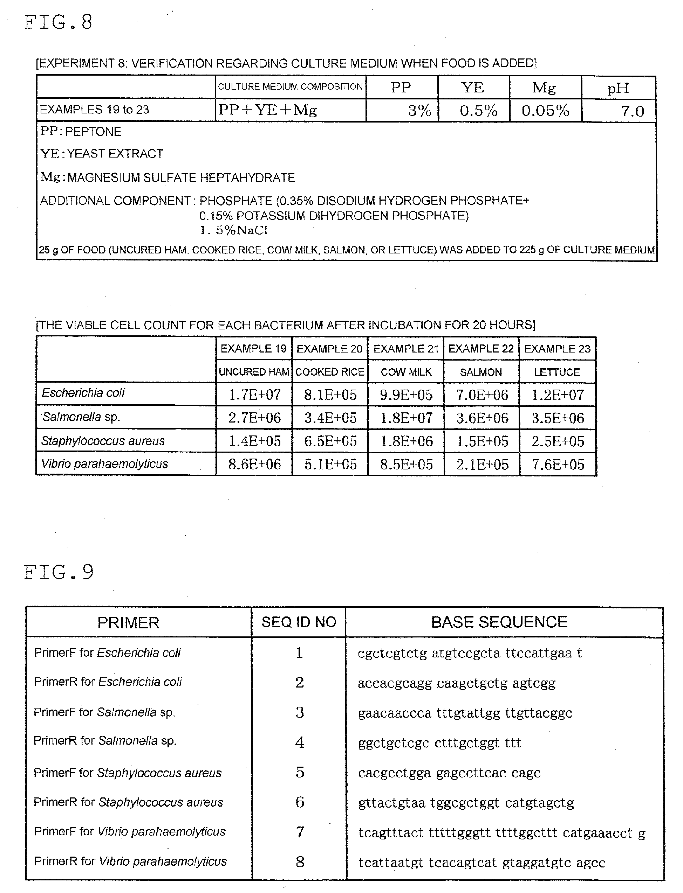

A PCR reaction mixture that includes a primer set that can specifically amplify the amplification target region of the DNA of *Escherichia coli, Salmonella* sp., *Staphylococcus aureus,* and *Vibrio parahaemolyticus* is prepared. The PCR reaction mixture may include a buffer, a nucleic acid substrate (dATP, dCTP, dGTP, and dTTP), a primer set (forward primer and reverse primer), a nucleic acid polymerase, the DNA of the food poisoning bacteria, and water. The primer set for *Escherichia coli* may preferably include a primer having a base sequence corresponding to SEQ ID NO: 1 and a primer having a base sequence corresponding to SEQ ID NO: 2, the primer set for *Salmonella* sp. may preferably include a primer having a base sequence corresponding to SEQ ID NO: 3 and a primer having a base sequence corresponding to SEQ ID NO: 4, the primer set for *Staphylococcus aureus* may preferably include a primer having a base sequence corresponding to SEQ ID NO: 5 and a primer having a base sequence corresponding to SEQ ID NO: 6, and the primer set for *Vibrio parahaemolyticus* may preferably include a primer having a base sequence corresponding to SEQ ID NO: 7 and a primer having a base sequence corresponding to SEQ ID NO: 8 (see FIG. 9).

PCR gene amplification may be performed using a PCR device (e.g., thermal cycler) under the following conditions, for example.
(1) 95°C, 2 min
(2) 95°C, 10 sec (DNA strand separation step (denaturation step))
(3) 68°C, 30 sec (annealing step)
(4) 72°C, 30 sec (DNA synthesis step)
(5) 72°C, 2 min

The steps (2) to (4) are repeated in 40 cycles.

The PCR amplified product is subjected to electrophoresis using a microcapillary or the like to check whether or not a correct amplified product has been obtained.

The base sequence length of the amplified product estimated for *Escherichia coli* is 158 bp, the base sequence length of the amplified product estimated for *Salmonella* sp. is 177 bp, the base sequence length of the amplified product estimated for *Staphylococcus aureus* is 238 bp, and the base sequence length of the amplified product estimated for *Vibrio parahaemolyticus* is 380 bp. When subjecting the PCR amplified product to electrophoresis, the base sequence of the PCR amplified product may not completely coincide with the theoretical base sequence length (i.e., approximate results are obtained) due to the effects of a reagent used for the electrophoresis device and the like.

Note that whether or not a correct amplified product has been obtained may be determined using a DNA chip, a DNA microarray, or the like instead of performing electrophoresis. In such a case, whether or not a correct amplified product has been obtained may be determined using a normal method.

The culture medium for food poisoning bacteria according to the embodiments of the invention makes it possible to allow *Escherichia coli, Salmonella* sp., *Staphylococcus aureus,* and *Vibrio parahaemolyticus* to grow at the same time in a single culture medium, and thereby allow all of *Escherichia coli, Salmonella* sp., *Staphylococcus aureus,* and *Vibrio parahaemolyticus* to grow to reach the detectable level (i.e., a bacterial count sufficient for detection) within a relatively short time. More specifically, it is possible to allow the food poisoning bacteria (respective concentration: 1 cfu/ml) to grow to reach a concentration of 10⁵ cfu/ml or more within 20 hours. It is also possible to allow the food poisoning bacteria to grow so that the difference between the maximum bacterial count and the minimum bacterial count is less than 10³ cfu/ml.

Therefore, the method for detecting food poisoning bacteria according to the embodiments of the invention can accurately detect a plurality of types of food poisoning bacteria that have been simultaneously incubated using the culture medium for food poisoning bacteria according to the embodiments of the invention.

### EXAMPLES

Experiment 1: Verification regarding culture medium including peptone, yeast extract, and magnesium sulfate

*Escherichia coli, Salmonella* sp., *Staphylococcus aureus,* and *Vibrio parahaemolyticus* were simultaneously incubated using a culture medium including a peptone, a yeast extract, and magnesium sulfate (Example 1), or a culture medium including only one component or two components among a peptone, a yeast extract, and magnesium sulfate (Comparative Examples 1 to 4), and the viable cell count for each bacterium after incubation for 20 hours at 37°C, was counted.

Specifically, a peptone, a yeast extract, magnesium sulfate, sodium chloride, disodium hydrogen phosphate, and potassium dihydrogen phosphate were mixed in the amounts shown below, and the pH of the mixture was adjusted to 7.0. Note that the balance was sterilized water (hereinafter the same). The resulting culture medium was sterilized at 121°C for 15 minutes using an autoclave.

### (Amount per L)

Peptone: 0 g or 10 g
Yeast extract: 0 g or 5 g
Magnesium sulfate heptahydrate: 0 g or 0.5 g
Sodium chloride: 15 g
Disodium hydrogen phosphate: 3.5 g
Potassium dihydrogen phosphate: 1.5 g

The following strains were respectively used as *Escherichia coli, Salmonella* sp., *Staphylococcus aureus,* and *Vibrio parahaemolyticus.*

### Escherichia coli: Escherichia coli NCTC 12923

*Salmonella* sp.: *Salmonella enterica* subsp. *Enterica* serovar Abony NCTC 6017
*Staphylococcus aureus: Staphylococcus aureus* NCTC 10788
*Vibrio parahaemolyticus: Vibrio parahaemolyticus* RIMD 2210050

These food poisoning bacteria strains were transferred from the following organizations (hereinafter the same).
NCTC: National Collection of Type Cultures (U.K.)
RIMD: Research Institute for Microbial Diseases, Osaka University

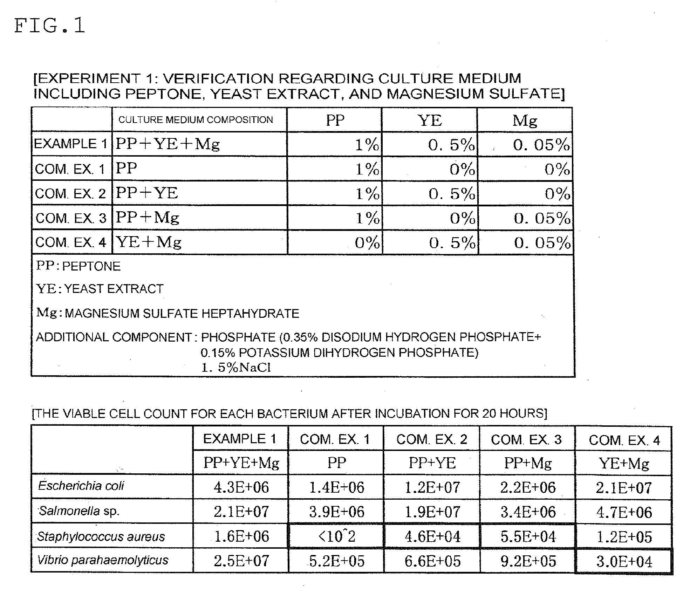

The four strains were simultaneously inoculated into 250 mL of each culture medium (i.e., the culture media of Example 1 and Comparative Examples 1 to 4) in an amount of 10 to 50 cfu, respectively. The bacteria were incubated at 37°C for 20 hours, and each bacterial count was counted. The viable cell count for *Escherichia coli* was counted using Compact Dry EC (manufactured by Nissui Pharmaceutical Co., Ltd.) as a selective medium, the viable cell count for *Salmonella* sp. was counted using a DHL agar medium (manufactured by Kyokuto Pharmaceutical Industrial Co., Ltd.) as a selective medium, the viable cell count for *Staphylococcus aureus* was counted using a mannitol salt agar medium with egg yolk (manufactured by Kyokuto Pharmaceutical Industrial Co., Ltd.) as a selective medium, and the viable cell count for *Vibrio parahaemolyticus* was counted using a TCBS agar medium (manufactured by Kyokuto Pharmaceutical Industrial Co., Ltd.) as a selective medium. A serial diluent of the culture was added to each selective medium. After culturing the bacteria at 35°C for 20 to 24 hours (note that *Staphylococcus aureus* was incubated for 48 hours), the typical colony of each type of bacteria was counted. FIG. 1 shows the culture medium composition, and the bacterial count after completion of culture.

As shown in FIG. 1, the *Escherichia coli* count, the *Salmonella* sp. count, the *Staphylococcus aureus* count, and the *Vibrio parahaemolyticus* count increased to 10⁶ or more when using the culture medium of Example 1 including a peptone, a yeast extract, and magnesium sulfate. On the other hand, when using the culture medium that did not include at least either a yeast extract or magnesium sulfate (Comparative Examples 1 to 3), the *Staphylococcus aureus* count was less than 10⁵ (i.e., *Staphylococcus aureus* did not grow to the detectable level). When using the culture medium that did not include a peptone (Comparative Example 4), the *Vibrio parahaemolyticus* count was less than 10⁵ (i.e., *Vibrio parahaemolyticus* did not grow sufficiently).

It was thus confirmed that it is possible to satisfactorily allow *Escherichia coli, Salmonella* sp., *Staphylococcus aureus,* and *Vibrio parahaemolyticus* to grow at the same time in a single culture medium by incorporating a peptone, a yeast extract, and magnesium sulfate in the culture medium.

### Experiment 2: Verification regarding optimum peptone concentration in culture medium

Whether or not it is possible to satisfactorily allow *Escherichia coli, Salmonella* sp., *Staphylococcus aureus,* and *Vibrio parahaemolyticus* to grow at the same time was determined using culture media differing in peptone concentration.

Specifically, a peptone, a yeast extract, magnesium sulfate, sodium chloride, disodium hydrogen phosphate, and potassium dihydrogen phosphate were mixed in the amounts shown below, and the pH of the mixture was adjusted to 7.0 to prepare a culture medium. The resulting culture medium was sterilized at 121°C for 15 minutes using an autoclave.

### (Amount per L)

Peptone: 0 g, 10 g, 20 g, 30 g, 40 g, or 50 g
Yeast extract: 5 g
Magnesium sulfate heptahydrate: 0.5 g
Sodium chloride: 15 g
Disodium hydrogen phosphate: 3.5 g
Potassium dihydrogen phosphate: 1.5 g

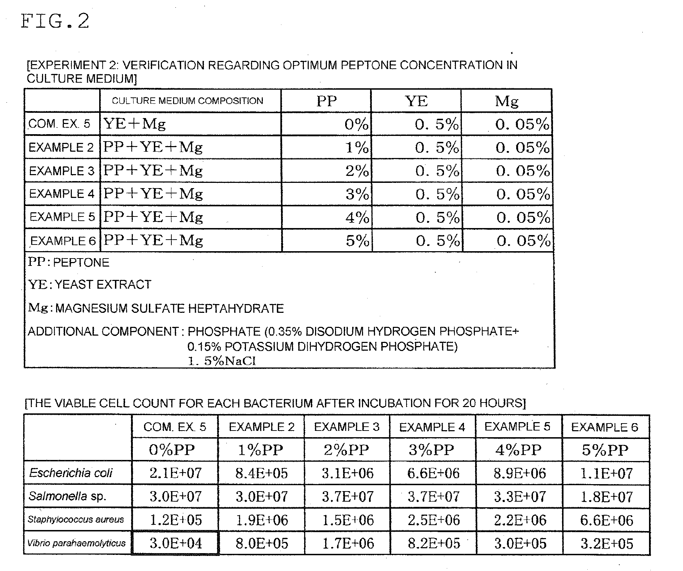

The same food poisoning bacteria *(Escherichia coli, Salmonella* sp., *Staphylococcus aureus,* and *Vibrio parahaemolyticus)* as those used in Experiment 1 were simultaneously incubated, and the viable cell count for each bacterium was counted in the same manner as in Experiment 1. FIG. 2 shows the culture medium composition, and the bacterial count after completion of culture.

As shown in FIG. 2, when the peptone concentration in the culture medium was 0%, the *Vibrio parahaemolyticus* count was less than 10⁵ (i.e., *Vibrio parahaemolyticus* did not grow sufficiently). On the other hand, the *Escherichia coli* count, the *Salmonella* sp. count, the *Staphylococcus aureus* count, and the *Vibrio parahaemolyticus* count increased to 10⁵ or more when the peptone concentration in the culture medium was 1% w/v or more.

It was thus confirmed that it is possible to satisfactorily allow *Escherichia coli, Salmonella* sp., *Staphylococcus aureus,* and *Vibrio parahaemolyticus* to grow at the same time in a single culture medium by incorporating a peptone in the culture medium at a concentration of 1% w/v or more.

### Experiment 3: Verification regarding optimum sodium chloride concentration in culture medium

Whether or not it is possible to satisfactorily allow *Escherichia coli, Salmonella* sp., *Staphylococcus aureus,* and *Vibrio parahaemolyticus* to grow at the same time was determined using culture media including a peptone, a yeast extract, and magnesium sulfate, and differing in sodium chloride concentration.

Specifically, a peptone, a yeast extract, magnesium sulfate, sodium chloride, disodium hydrogen phosphate, and potassium dihydrogen phosphate were mixed in the amounts shown below, and the pH of the mixture was adjusted to 7.0 to prepare a culture medium. The resulting culture medium was sterilized at 121°C for 15 minutes using an autoclave.

### (Amount per L)

Peptone: 10 g
Yeast extract: 5 g
Magnesium sulfate heptahydrate: 0.5 g
Sodium chloride: 0 g, 5 g, 10 g, 15 g, 20 g, 25 g, 30 g, or 35 g
Disodium hydrogen phosphate: 3.5 g
Potassium dihydrogen phosphate: 1.5 g

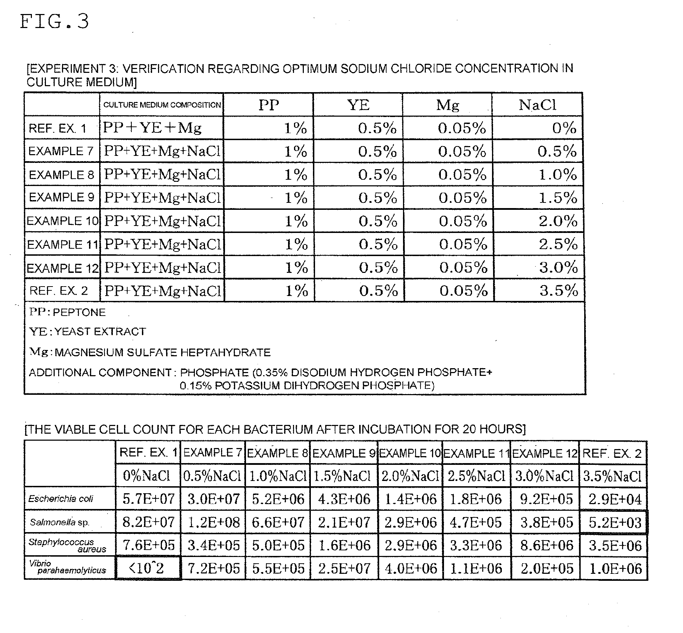

The same food poisoning bacteria *(Escherichia coli, Salmonella* sp., *Staphylococcus aureus,* and *Vibrio parahaemolyticus)* as those used in Experiment 1 were simultaneously incubated, and the viable cell count for each bacterium was counted in the same manner as in Experiment 1.. FIG. 3 shows the culture medium composition, and the bacterial count after completion of culture.

As shown in FIG. 3, when the sodium chloride concentration in the culture medium was 0% w/v, the *Vibrio parahaemolyticus* count was less than 10² (i.e., *Vibrio parahaemolyticus* grew to only a small extent). On the other hand, the *Escherichia coli* count, the *Salmonella* sp. count, the *Staphylococcus aureus* count, and the *Vibrio parahaemolyticus* count increased to 10⁵ or more when the sodium chloride concentration in the culture medium was 0.5 to 3.0% w/v. When the sodium chloride concentration in the culture medium was 3.5% w/v, the *Salmonella* sp. count was less than 10⁴ (i.e., *Salmonella* sp. did not grow sufficiently).

It was thus confirmed that it is possible to satisfactorily allow *Escherichia coli, Salmonella* sp., *Staphylococcus aureus,* and *Vibrio parahaemolyticus* to grow at the same time in a single culture medium by incorporating sodium chloride in the culture medium at a concentration of 0.5% w/v to 3.0% w/v. In particular, *Staphylococcus aureus* and *Vibrio parahaemolyticus* grew sufficiently when the sodium chloride concentration was 1.5% w/v to 2.5% w/v. When the sodium chloride concentration was 1.5% w/v to 3.0% w/v, the *Escherichia coli* count, the *Salmonella* sp. count, the *Staphylococcus aureus* count, and the *Vibrio parahaemolyticus* count increased to 10⁶ or more (i.e., it was possible to allow *Escherichia coli, Salmonella* sp., *Staphylococcus aureus,* and *Vibrio parahaemolyticus* to grow in a particularly well-balanced manner).

### Experiment 4: Verification regarding optimum glucose concentration in culture medium

Whether or not it is possible to satisfactorily allow *Escherichia coli, Salmonella* sp., *Staphylococcus aureus,* and *Vibrio parahaemolyticus* to grow at the same time was determined using culture media including a peptone, a yeast extract, and magnesium sulfate, and differing in glucose concentration.

Specifically, a peptone, a yeast extract, magnesium sulfate, sodium chloride, disodium hydrogen phosphate, potassium dihydrogen phosphate, and glucose were mixed in the amounts shown below, and the pH of the mixture was adjusted to 7.0 to prepare a culture medium. The resulting culture medium was sterilized at 121°C for 15 minutes using an autoclave.

### (Amount per L)

Peptone: 30 g
Yeast extract: 5 g
Magnesium sulfate heptahydrate: 0.5 g
Sodium chloride: 15 g
Disodium hydrogen phosphate: 3.5 g
Potassium dihydrogen phosphate: 1.5 g
Glucose: 0 g, 5 g, or 10 g

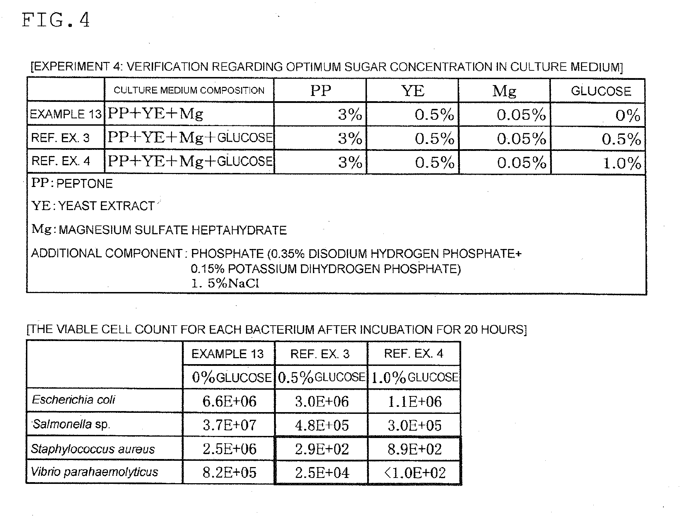

The same food poisoning bacteria *(Escherichia coli, Salmonella* sp., *Staphylococcus aureus,* and *Vibrio parahaemolyticus)* as those used in Experiment 1 were simultaneously incubated, and the viable cell count for each bacterium was counted in the same manner as in Experiment 1.. FIG. 4 shows the culture medium composition, and the bacterial count after completion of culture.

As shown in FIG. 4, the *Escherichia coli* count, the *Salmonella* sp. count, the *Staphylococcus aureus* count, and the *Vibrio parahaemolyticus* count increased to 10⁵ or more when the glucose concentration in the culture medium was 0% w/v. On the other hand, *Staphylococcus aureus* and *Vibrio parahaemolyticus* did not grow sufficiently when the glucose concentration in the culture medium was 0.5% w/v or 1.0% w/v. It is considered that the doubling time increased when the glucose concentration in the culture medium was high, and *Escherichia coli* and *Salmonella* sp. grew rapidly, and underwent metabolism and fermentation to produce an organic acid and the like, whereby the growth of *Staphylococcus aureus* and *Vibrio parahaemolyticus* was inhibited.

It was thus confirmed that glucose (that is normally added to a culture medium in order to promote growth) inhibits the growth of *Staphylococcus aureus* and *Vibrio parahaemolyticus* when culturing *Staphylococcus aureus* and *Vibrio parahaemolyticus* in a single culture medium together with *Escherichia coli* and *Salmonella* sp.

Therefore, it is preferable that the culture medium according to the embodiments of the invention not include glucose.

### Experiment 5: Verification regarding optimum pH of culture medium

Whether or not it is possible to satisfactorily allow *Escherichia coli, Salmonella* sp., *Staphylococcus aureus,* and *Vibrio parahaemolyticus* to grow at the same time was determined using culture media including a peptone, a yeast extract, and magnesium sulfate, and differing in pH.

Specifically, a peptone, a yeast extract, magnesium sulfate, sodium chloride, disodium hydrogen phosphate, and potassium dihydrogen phosphate were mixed in the amounts shown below, and the pH of the mixture was adjusted to 6.0, 6.5, 7.0, 7.5, or 8.0 to prepare a culture medium. The resulting culture medium was sterilized at 121°C for 15 minutes using an autoclave.

### (Amount per L)

Peptone: 30 g
Yeast extract: 5 g
Magnesium sulfate heptahydrate: 0.5 g
Sodium chloride: 15 g
Disodium hydrogen phosphate: 3.5 g
Potassium dihydrogen phosphate: 1.5 g
pH: 6.0, 6.5, 7.0, 7.5, or 8.0

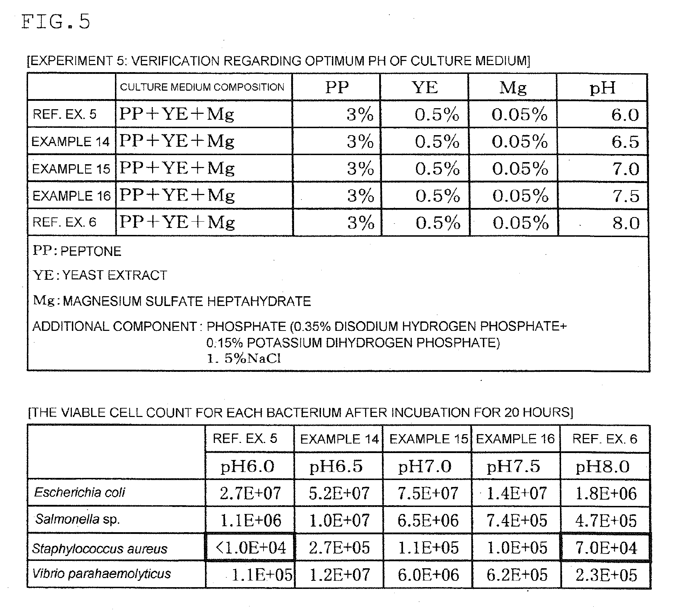

The same food poisoning bacteria *(Escherichia coli, Salmonella* sp., *Staphylococcus aureus,* and *Vibrio parahaemolyticus)* as those used in Experiment 1 were simultaneously incubated, and the viable cell count for each bacterium was counted in the same manner as in Experiment 1. FIG. 5 shows the culture medium composition, and the bacterial count after completion of culture.

As shown in FIG. 5, when the pH of the culture medium was 6.0, the *Staphylococcus aureus* count was less than 10⁵ (i.e., *Staphylococcus aureus* did not grow sufficiently). On the other hand, the *Escherichia coli* count, the *Salmonella* sp. count, the *Staphylococcus aureus* count, and the *Vibrio parahaemolyticus* count increased to 10⁵ or more when the pH of the culture medium was 6.5 to 7.5. When the pH of the culture medium was 8.0, the *Staphylococcus aureus* count was less than 10⁵ (i.e., *Staphylococcus aureus* did not grow sufficiently).

It was thus confirmed that it is possible to satisfactorily allow *Escherichia coli, Salmonella* sp., *Staphylococcus aureus,* and *Vibrio parahaemolyticus* to grow at the same time in a single culture medium by adjusting the pH of the culture medium to 6.5 to 7.5.

### Experiment 6: Verification regarding bacterial count after completion of culture using culture medium according to the embodiments of the invention and existing culture medium

Whether or not it is possible to satisfactorily allow *Escherichia coli, Salmonella* sp., *Staphylococcus aureus,* and *Vibrio parahaemolyticus* to grow at the same time was determined using the culture medium for food poisoning bacteria according to the embodiments of the invention and an existing culture medium for food poisoning bacteria (medium No. 17 of Patent Document 2 ("TA10 Broth" manufactured by Prima Meat Packers, Ltd.)).

The culture medium for food poisoning bacteria according to the embodiments of the invention and the existing culture medium for food poisoning bacteria had the following composition. Each culture medium was sterilized at 121°C for 15 minutes using an autoclave.

### Culture medium for food poisoning bacteria according to the embodiments of the invention

### (Amount per L)

Peptone: 30 g
Yeast extract: 5 g
Magnesium sulfate heptahydrate: 0.5 g
Sodium chloride: 15 g
Disodium hydrogen phosphate: 3.5 g
Potassium dihydrogen phosphate: 1.5 g pH: 7.0

### Existing culture medium for food poisoning bacteria

### (Amount per L)

Peptone: 10 g
Yeast extract: 5 g
Magnesium sulfate heptahydrate: 0 g
Sodium chloride: 5 g
Disodium hydrogen phosphate: 7 g
Potassium dihydrogen phosphate: 15 g
Meat extract: 5 g
Glucose: 0.5 g
Salts: 0.95 g
pH: 6.3

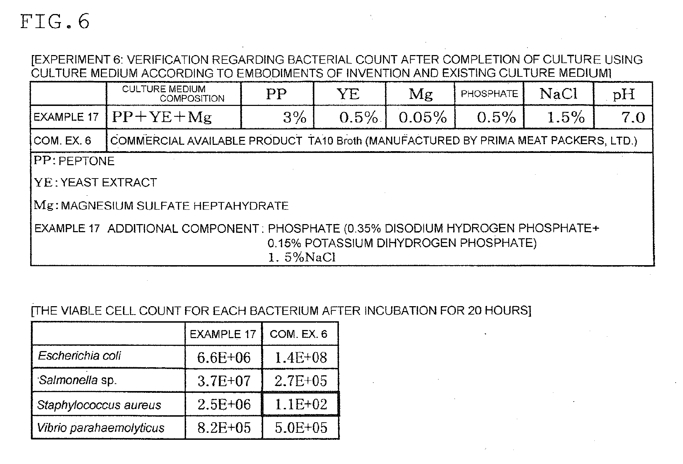

The same food poisoning bacteria *(Escherichia coli, Salmonella* sp., *Staphylococcus aureus,* and *Vibrio parahaemolyticus)* as those used in Experiment 1 were simultaneously incubated, and the viable cell count for each bacterium was counted in the same manner as in Experiment 1. FIG. 6 shows the culture medium composition, and the bacterial count after completion of culture.

As shown in FIG. 6, the *Escherichia coli* count, the *Salmonella* sp. count, the *Staphylococcus aureus* count, and the *Vibrio parahaemolyticus* count increased to 10⁵ or more when the culture medium for food poisoning bacteria according to the embodiments of the invention was used. On the other hand, *Staphylococcus aureus* grew to only a small extent when the existing culture medium for food poisoning bacteria was used.

It was thus confirmed that it is impossible to sufficiently allow *Staphylococcus aureus* to grow when culturing *Escherichia coli, Salmonella* sp., *Staphylococcus aureus,* and *Vibrio parahaemolyticus* at the same time using the existing culture medium for food poisoning bacteria, while it is possible to satisfactorily allow *Escherichia coli, Salmonella* sp., *Staphylococcus aureus,* and *Vibrio parahaemolyticus* to grow at the same time in a single culture medium by utilizing the culture medium for food poisoning bacteria according to the embodiments of the invention.

### Experiment 7: Verification regarding culture medium when contaminating bacteria are mixed

Whether or not it is possible to satisfactorily allow *Escherichia coli, Salmonella* sp., *Staphylococcus aureus,* and *Vibrio parahaemolyticus* to grow at the same time was determined using a culture medium including a peptone, a yeast extract, and magnesium sulfate, into which *Bacillus subfilis* was inoculated as contaminating bacteria in an amount of 1.2×10⁷ cfu. The same culture medium as the culture medium for food poisoning bacteria according to the embodiments of the invention used in Experiment 6 was used as the culture medium.

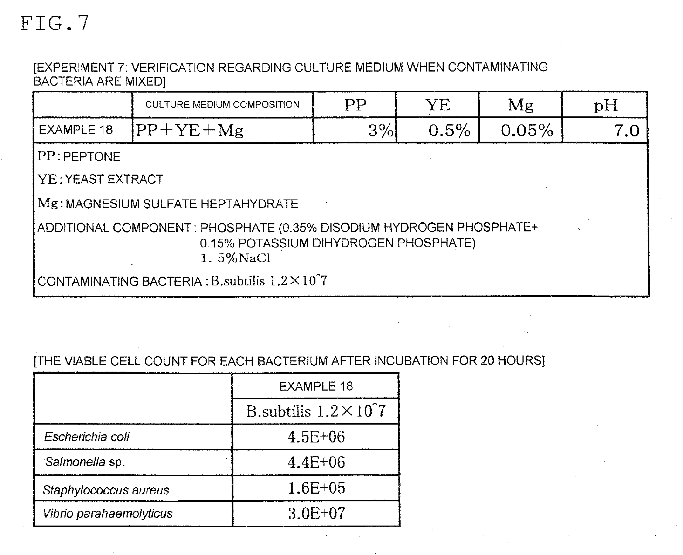

The same strains of *Escherichia coli, Salmonella* sp., *Staphylococcus aureus,* and *Vibrio parahaemolyticus* as those used in Experiment 1 were used as *Escherichia coli, Salmonella* sp., *Staphylococcus aureus,* and *Vibrio parahaemolyticus.* These food poisoning bacteria were inoculated into the culture medium in an amount of 10 to 50 cfu, and *Bacillus subtilis* was also inoculated into the culture medium. The food poisoning bacteria were simultaneously incubated in the presence of *Bacillus subtilis* (i.e., contaminating bacteria), and the viable cell count for each bacterium was counted in the same manner as in Experiment 1. FIG. 7 shows the culture medium composition, and the bacterial count after completion of culture.

As shown in FIG. 7, the *Escherichia coli* count, the *Salmonella* sp. count, the *Staphylococcus aureus* count, and the *Vibrio parahaemolyticus* count increased to 10⁵ or more when the culture medium for food poisoning bacteria according to the embodiments of the invention was used.

It was thus confirmed that it is possible to satisfactorily allow *Escherichia coli, Salmonella* sp., *Staphylococcus aureus,* and *Vibrio parahaemolyticus* to grow at the same time in a single culture medium even when contaminating bacteria are mixed, by utilizing the culture medium for food poisoning bacteria according to the embodiments of the invention.

### Experiment 8: Verification regarding culture medium when food is added

When food is added to a culture medium, the bacterial count (microbial count) after completion of culture normally decreases due to the effects of food contaminants and contaminating bacteria. Whether or not it is possible to allow *Escherichia coli, Salmonella* sp., *Staphylococcus aureus,* and *Vibrio parahaemolyticus* to sufficiently grow at the same time in the culture medium according to the embodiments of the invention even when food is added to the culture medium, was determined.

The same culture medium as the culture medium for food poisoning bacteria according to the embodiments of the invention used in Experiment 6 was used as the culture medium.

The same strains of *Escherichia coli, Salmonella* sp., *Staphylococcus aureus,* and *Vibrio parahaemolyticus* as those used in Experiment 1 were used as *Escherichia coli, Salmonella* sp., *Staphylococcus aureus,* and *Vibrio parahaemolyticus.* Uncured ham, cooked rice, cow milk, salmon, and lettuce were independently used as the food.

25 g of the food that was cut finely was added to 225 g of the culture medium. After inoculating *Escherichia coli, Salmonella* sp., *Staphylococcus aureus,* and *Vibrio parahaemolyticus* into the culture medium in an amount of 10 to 50 cfu, the mixture was mixed for 60 seconds using a stomacher. The mixture was simultaneously incubated at 37°C for 20 hours, and the viable cell count for each bacterium was counted in the same manner as in Experiment 1. FIG. 8 shows the culture medium composition, and the bacterial count after completion of culture.

As shown in FIG. 8, the *Escherichia coli* count, the *Salmonella* sp. count, the *Staphylococcus aureus* count, and the *Vibrio parahaemolyticus* count increased to 10⁵ or more when the culture medium for food poisoning bacteria according to the embodiments of the invention to which each food was added was used.

It was thus confirmed that it is possible to satisfactorily allow *Escherichia coli, Salmonella* sp., *Staphylococcus aureus,* and *Vibrio parahaemolyticus* to grow at the same time in a single culture medium even when uncured ham, cooked rice, cow milk, salmon, or lettuce is added to the culture medium.

### Experiment 9: Verification (1) regarding detection of food poisoning bacteria using rapid detection method

*Escherichia coli, Salmonella* sp., *Staphylococcus aureus,* and *Vibrio parahaemolyticus* were simultaneously incubated at 37°C for 20 hours using the culture media of Example 1 and Comparative Examples 1 to 3 (see Experiment 1). The food poisoning bacteria that had grown were mixed of the culture media respectively, and DNA was extracted.

The amplification target region of each type of food poisoning bacteria was grown at the same time by multiplex PCR using primers for specifically amplifying the amplification target region of the DNA of each type of food poisoning bacteria, and whether or not the resulting amplified product can be detected was determined. Specifically, the following operation was performed.

1 mL of each culture medium was collected after completion of culture, and centrifuged at 5000xg for 10 minutes. After removing the supernatant liquid, a lysozyme aqueous solution (concentration: 20 mg/ml) (20 mM Tris-HCl, pH: 8.0/2 mM EDTA, 1.2% Triton X-100) was added to the precipitate, and a bacteriolysis treatment was performed at 37°C for 30 minutes. Column purification was then performed using a DNeasy Blood & Tissue Kit (manufactured by Qiagen) to obtain a DNA extract. The DNA extract was used as a sample subjected to multiplex PCR.

A PCR reaction mixture having the composition shown below was prepared. Each primer was synthesized by Sigma-Aldrich Co. LLC. on commission, and the other reagents are products manufactured by Takara Bio Inc.

Buffer (10×Ex Taq buffer): 2.0 µl
Nucleic acid substrate (dNTP Mixture): 1.6 µl
PrimerF for *Escherichia coli* (SEQ ID NO: 1): 0.2 µl
PrimerR for *Escherichia coli* (SEQ ID NO: 2): 0.2 µl
PrimerF for *Salmonella* sp. (SEQ ID NO: 3): 0.2 µl
PrimerR for *Salmonella* sp. (SEQ ID NO: 4): 0.2 µl
PrimerF for *Staphylococcus aureus* (SEQ ID NO: 5): 0.2 µl
PrimerR for *Staphylococcus aureus* (SEQ ID NO: 6): 0.2 µl
PrimerF for *Vibrio parahaemolyticus* (SEQ ID NO: 7): 0.2 µl
PrimerR for *Vibrio parahaemolyticus* (SEQ ID NO: 8): 0.2 µl
TaKaRa Ex Taq Hot Start Version: 0.1 µl
DNA sample: 0.1 µl
Sterilized water: 13.7 µl

### (Total amount: 20 µl)

PCR gene amplification was performed using a system "Thermal Cycler ep gradient" (manufactured by Eppendorf). The reactive conditions are shown below.
(1) 95°C, 2 min
(2) 95°C, 10 sec (DNA strand separation step (denaturation step))
(3) 68°C, 10 sec (annealing step)
(4) 72°C, 30 sec (DNA synthesis step)
(5) 72°C, 2 min

The steps (2) to (4) were repeated in 40 cycles.

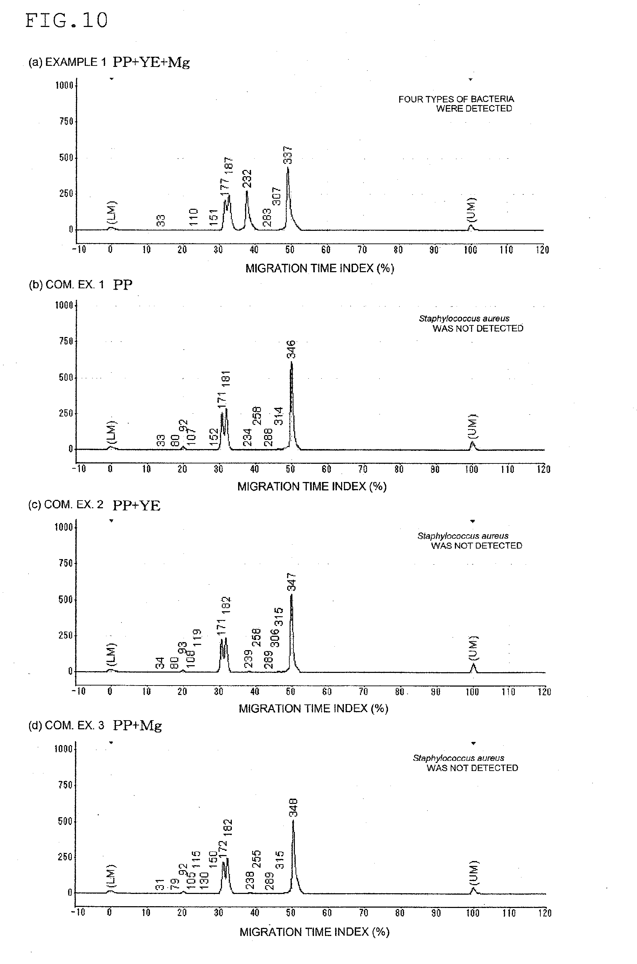

The PCR reaction mixture was analyzed using a microchip electrophoresis device "MultiNA" (manufactured by Shimadzu Corporation) to determine the size of the PCR amplified product. The results are shown in FIG. 10.

As shown in FIG. 10, when *Escherichia coli, Salmonella* sp., *Staphylococcus aureus,* and *Vibrio parahaemolyticus* were simultaneously incubated using the culture medium of Example 1 including a peptone, a yeast extract, and magnesium sulfate, all of *Escherichia coli, Salmonella* sp., *Staphylococcus aureus,* and *Vibrio parahaemolyticus* could be sufficiently detected. In the graph illustrated in FIG. 10 (see (a)), the peak corresponding to an estimated base length of 177 corresponds to *Escherichia coli,* the peak corresponding to an estimated base length of 187 corresponds to *Salmonella* sp., the peak corresponding to an estimated base length of 232 corresponds to *Staphylococcus aureus,* and the peak corresponding to an estimated base length of 337 corresponds to *Vibrio parahaemolyticus.*

The base sequence length of the amplified product estimated for *Escherichia coli* is 157 bp, the base sequence length of the amplified product estimated for *Salmonella* sp. is 180 bp, the base sequence length of the amplified product estimated for *Staphylococcus aureus* is 236 bp, and the base sequence length of the amplified product estimated for *Vibrio parahaemolyticus* is 380 bp. It is considered that the difference between the estimated base length and the base sequence length of the amplified product by about several bp to about several tens of bp occurred due to an error during electrophoresis.

When *Escherichia coli, Salmonella* sp., *Staphylococcus aureus,* and *Vibrio parahaemolyticus* were simultaneously incubated using the culture medium of Comparative Example 1 that included a peptone, and did not include a yeast extract and magnesium sulfate, *Staphylococcus aureus* could not be sufficiently detected. In the graph illustrated in FIG. 10 (see (b)), the peak corresponding to an estimated base length of 171 corresponds to *Escherichia coli,* the peak corresponding to an estimated base length of 181 corresponds to *Salmonella* sp., and the peak corresponding to an estimated base length of 346 corresponds to *Vibrio parahaemolyticus.* However, a peak corresponding to *Staphylococcus aureus* was not observed.

When *Escherichia coli, Salmone0a* sp., *Staphylococcus aureus,* and *Vibrio parahaemolyticus* were simultaneously incubated using the culture medium of Comparative Example 2 that included a peptone and a yeast extract, and did not include magnesium sulfate, *Staphylococcus aureus* could not be sufficiently detected. In the graph illustrated in FIG. 10 (see (c)), the peak corresponding to an estimated base length of 171 corresponds to *Escherichia coli,* the peak corresponding to an estimated base length of 182 corresponds to *Salmonella* sp., and the peak corresponding to an estimated base length of 347 corresponds to *Vibrio parahaemolyticus.* However, a peak corresponding to *Staphylococcus aureus* was not observed.

When *Escherichia coli, Salmonella* sp., *Staphylococcus aureus,* and *Vibrio parahaemolyticus* were simultaneously incubated using the culture medium of Comparative Example 3 that included a peptone and magnesium sulfate, and did not include a yeast extract, *Staphylococcus aureus* could not be sufficiently detected. In the graph illustrated in FIG. 10 (see (d)), the peak corresponding to an estimated base length of 172 corresponds to *Escherichia coli,* the peak corresponding to an estimated base length of 182 corresponds to *Salmonella* sp., and the peak corresponding to an estimated base length of 348 corresponds to *Vibrio parahaemolyticus.* However, a peak corresponding to *Staphylococcus aureus* was not observed.

It was thus confirmed that *Escherichia coli, Salmonella* sp., *Staphylococcus aureus,* and *Vibrio parahaemolyticus* can be sufficiently simultaneously incubated using the culture medium for food poisoning bacteria according to the embodiments of the invention that includes a peptone, a yeast extract, and magnesium sulfate, and all of *Escherichia coli, Salmonella* sp., *Staphylococcus aureus,* and *Vibrio parahaemolyticus* can be detected using the rapid detection method that utilizes multiplex PCR and electrophoresis.

### Experiment 10: Verification (2) regarding detection of food poisoning bacteria using rapid detection method

*Escherichia coli, Salmonella* sp., *Staphylococcus aureus,* and *Vibrio parahaemolyticus* were simultaneously incubated at 37°C for 20 hours using the culture media of Examples 7 to 12 and Reference Examples 1 and 2 (see Experiment 3). The food poisoning bacteria that had grown were mixed of the culture media respectively, and DNAwas extracted.

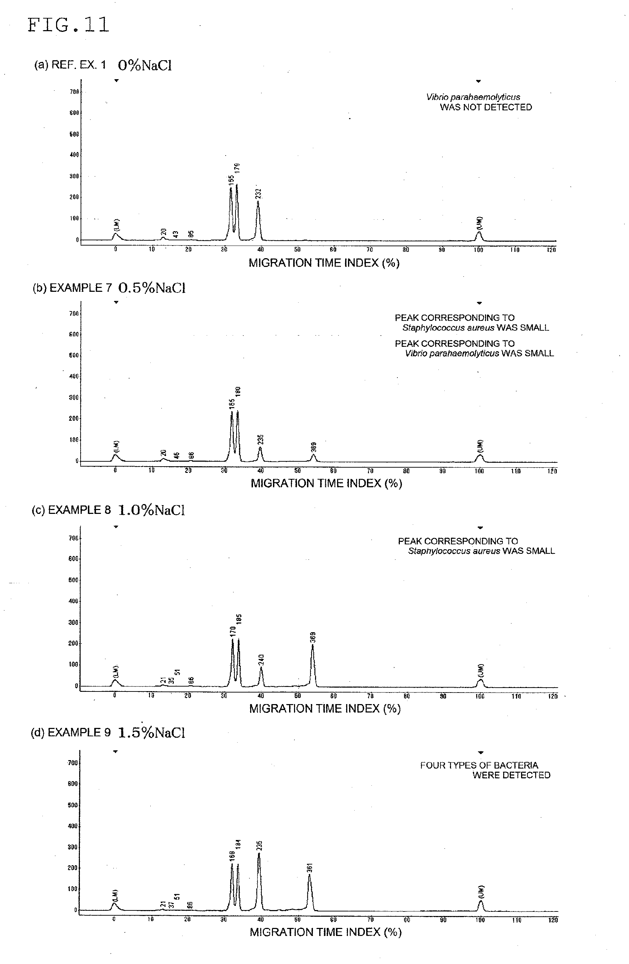

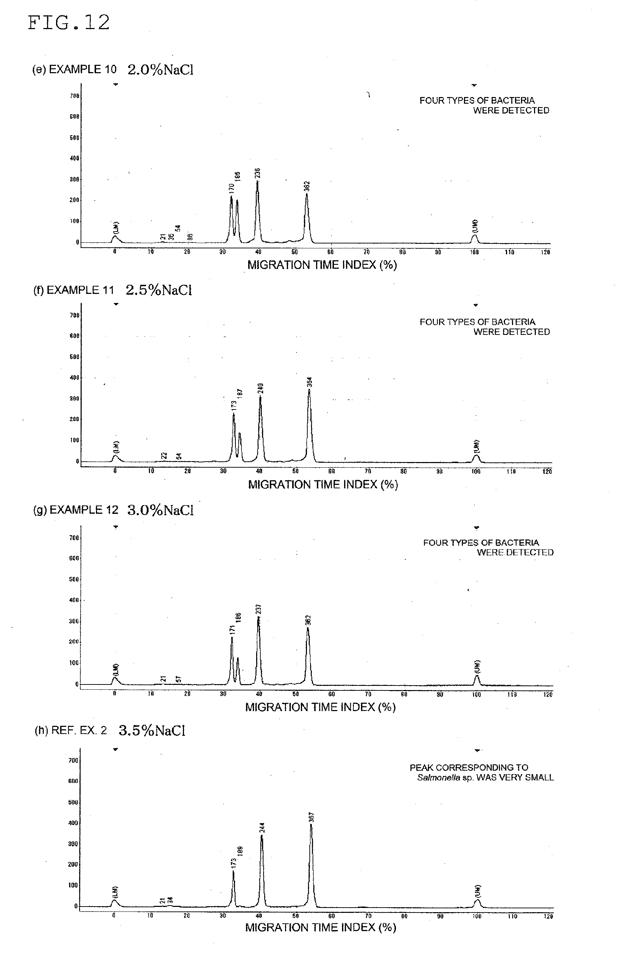

The amplification target region of each type of food poisoning bacteria was grown by multiplex PCR using primers for specifically amplifying the amplification target region of the DNA of each type of food poisoning bacteria, and whether or not the resulting amplified product can be detected was determined. The experiment was performed in the same manner as in Experiment 9, except that the culture medium was changed. The results are shown in FIGS. 11 and 12.

When *Escherichia coli, Salmonella* sp., *Staphylococcus aureus,* and *Vibrio parahaemolyticus* were incubated using the culture medium of Reference Example 1 having a sodium chloride concentration of 0%, *Vibrio parahaemolyticus* could not be sufficiently detected (see (a) in FIG. 11). In FIG. 11 (see (a)), the peak corresponding to an estimated base length of 165 corresponds to *Escherichia coli,* the peak corresponding to an estimated base length of 179 corresponds to *Salmonella* sp., and the peak corresponding to an estimated base length of 232 corresponds to *Staphylococcus aureus.* However, a peak corresponding to *Vibrio parahaemolyticus* was not observed.

When *Escherichia coli, Salmonella* sp., *Staphylococcus aureus,* and *Vibrio parahaemolyticus* were simultaneously incubated using the culture medium of Example 7 having a sodium chloride concentration of 0.5%, all of *Escherichia coli, Salmonella* sp., *Staphylococcus aureus,* and *Vibrio parahaemolyticus* could be detected (see (b) in FIG. 11). In FIG. 11 (see (b)), the peak corresponding to an estimated base length of 165 corresponds to *Escherichia coli,* and the peak corresponding to an estimated base length of 180 corresponds to *Salmonella* sp. The peak corresponding to an estimated base length of 235 corresponds to *Staphylococcus aureus,* and the peak corresponding to an estimated base length of 369 corresponds to *Vibrio parahaemolyticus.* Note that the peak corresponding to *Staphylococcus aureus* and the peak corresponding to *Vibrio parahaemolyticus* were smaller to some extent than the peak corresponding to *Escherichia coli* and the peak corresponding to *Salmonella* sp.

When *Escherichia coli, Salmonella* sp., *Staphylococcus aureus,* and *Vibrio parahaemolyticus* were simultaneously incubated using the culture medium of Example 8 having a sodium chloride concentration of 1.0%, all of *Escherichia coli, Salmonella* sp., *Staphylococcus aureus,* and *Vibrio parahaemolyticus* could be detected (see (c) in FIG. 11). In FIG. 11 (see (c)), the peak corresponding to an estimated base length of 170 corresponds to *Escherichia coli,* the peak corresponding to an estimated base length of 185 corresponds to *Salmonella* sp., and the peak corresponding to an estimated base length of 369 corresponds to *Vibrio parahaemolyticus.* The peak corresponding to an estimated base length of 240 corresponds to *Staphylococcus aureus.* Note that the peak corresponding to *Staphylococcus aureus* was smaller to some extent than the peak corresponding to *Escherichia coli,* the peak corresponding to *Salmonella* sp., and the peak corresponding to *Vibrio parahaemolyticus.*

When *Escherichia coli, Salmonella* sp., *Staphylococcus aureus,* and *Vibrio parahaemolyticus* were simultaneously incubated using the culture medium of Example 9 having a sodium chloride concentration of 1.5%, all of *Escherichia coli, Salmonella* sp., *Staphylococcus aureus,* and *Vibrio parahaemolyticus* could be detected (see (d) in FIG. 11). In FIG. 11 (see (d)), the peak corresponding to an estimated base length of 169 corresponds to *Escherichia coli,* the peak corresponding to an estimated base length of 184 corresponds to *Salmonella* sp., the peak corresponding to an estimated base length of 235 corresponds to *Staphylococcus aureus,* and the peak corresponding to an estimated base length of 361 corresponds to *Vibrio parahaemolyticus.*

When *Escherichia coli, Salmonella* sp., *Staphylococcus aureus,* and *Vibrio parahaemolyticus* were simultaneously incubated using the culture medium of Example 10 having a sodium chloride concentration of 2.0%, all of *Escherichia coli, Salmonella* sp., *Staphylococcus aureus,* and *Vibrio parahaemolyticus* could be detected (see (e) in FIG. 12). In FIG. 12 (see (e)), the peak corresponding to an estimated base length of 170 corresponds to *Escherichia coli,* the peak corresponding to an estimated base length of 185 corresponds to *Salmonella* sp., the peak corresponding to an estimated base length of 236 corresponds to *Staphylococcus aureus,* and the peak corresponding to an estimated base length of 362 corresponds to *Vibrio parahaemolyticus.*

When *Escherichia coli, Salmonella* sp., *Staphylococcus aureus,* and *Vibrio parahaemolyticus* were simultaneously incubated using the culture medium of Example 11 having a sodium chloride concentration of 2.5%, all of *Escherichia coli, Salmonella* sp., *Staphylococcus aureus,* and *Vibrio parahaemolyticus* could be detected (see (f) in FIG. 12). In FIG. 12 (see (f)), the peak corresponding to an estimated base length of 173 corresponds to *Escherichia coli,* the peak corresponding to an estimated base length of 187 corresponds to *Salmonella* sp., the peak corresponding to an estimated base length of 240 corresponds to *Staphylococcus aureus,* and the peak corresponding to an estimated base length of 364 corresponds to *Vibrio parahaemolyticus.*

When *Escherichia coli, Salmonella* sp., *Staphylococcus aureus,* and *Vibrio parahaemolyticus* were simultaneously incubated using the culture medium of Example 12 having a sodium chloride concentration of 3.0%, all of *Escherichia coli, Salmonella* sp., *Staphylococcus aureus,* and *Vibrio parahaemolyticus* could be detected (see (g) in FIG. 12). In FIG. 12 (see (g)), the peak corresponding to an estimated base length of 171 corresponds to *Escherichia coli,* the peak corresponding to an estimated base length of 186 corresponds to *Salmonella* sp., the peak corresponding to an estimated base length of 237 corresponds to *Staphylococcus aureus,* and the peak corresponding to an estimated base length of 362 corresponds to *Vibrio parahaemolyticus.*

When *Escherichia coli, Salmonella* sp., *Staphylococcus aureus,* and *Vibrio parahaemolyticus* were simultaneously incubated using the culture medium of Reference Example 2 having a sodium chloride concentration of 3.5%, *Salmonella* sp. could not be sufficiently detected (see (h) in FIG. 12). In FIG. 12 (see (h)), the peak corresponding to an estimated base length of 173 corresponds to *Escherichia coli,* the peak corresponding to an estimated base length of 244 corresponds to *Staphylococcus aureus,* and the peak corresponding to an estimated base length of 367 corresponds to *Vibrio parahaemolyticus.* The peak corresponding to an estimated base length of 189 corresponds to *Salmonella* sp. However, the peak corresponding to *Salmonella* sp. was very small.

Specifically, all of *Escherichia coli, Salmonella* sp., *Staphylococcus aureus,* and *Vibrio parahaemolyticus* could be particularly satisfactorily detected when the sodium chloride concentration was 1.5 to 3.0%.

It was thus confirmed that it is possible to allow *Escherichia coli, Salmonella* sp., *Staphylococcus aureus,* and *Vibrio parahaemolyticus* to sufficiently grow at the same time using the culture medium for food poisoning bacteria according to the embodiments of the invention, and all of *Escherichia coli, Salmonella* sp., *Staphylococcus aureus,* and *Vibrio parahaemolyticus* can be detected using the rapid detection method that utilizes multiplex PCR and electrophoresis.

The invention is not limited to the above embodiments and examples. Various modifications may be made without departing from the scope of the invention.

### INDUSTRIAL APPLICABILITY

The invention may suitably be used when it is desired to appropriately allow *Escherichia coli, Salmonella* sp., *Staphylococcus aureus,* and *Vibrio parahaemolyticus* to grow at the same time, and detect at least one of *Escherichia coli, Salmonella* sp., *Staphylococcus aureus,* and *Vibrio parahaemolyticus* in the fields of food inspection, epidemiological environmental inspection, environmental inspection, clinical examination, and livestock health management.

## Claims

1. A culture medium comprising a peptone, a yeast extract, and magnesium sulfate.

2. The culture medium according to claim 1, further comprising sodium chloride at a concentration of 1.5% w/v to 3.0% w/v.

3. The culture medium according to claim 1 or 2, not comprising glucose.

4. The culture medium according to any one of claims 1 to 3, comprising the peptone at a concentration of 1.0% w/v or more, further comprising disodium hydrogen phosphate and potassium dihydrogen phosphate, and having a pH of 6.5 to 7.5.

5. The culture medium according to any one of claims 1 to 4, the culture medium being used to allow a plurality of types of food poisoning bacteria including at least *Staphylococcus aureus* to grow at the same time.

6. The culture medium according to any one of claims 1 to 5, the culture medium being used to allow *Escherichia coli, Salmonella* sp., *Staphylococcus aureus,* and *Vibrio parahaemolyticus* to grow at the same time.

7. The culture medium according to any one of claims 1 to 6, the culture medium allowing 1 cfu/ml of each of a plurality of types of food poisoning bacteria to grow at the same time to a concentration of 10⁵ cfu/ml or more within 20 hours.

8. The culture medium according to any one of claims 1 to 7, the culture medium allowing 1 cfu/ml of each of a plurality of types of food poisoning bacteria to grow at the same time within 20 hours so that a difference between a maximum bacterial count and a minimum bacterial count is less than 10³ cfu/ml.

9. A method for detecting food poisoning bacteria comprising allowing a plurality of types of food poisoning bacteria including at least *Staphylococcus aureus* to grow at the same time using a culture medium that comprises a peptone, a yeast extract, and magnesium sulfate, extracting DNA from the plurality of types of food poisoning bacteria that have been allowed to grow, adding a primer for specifically amplifying an amplification target region of the DNA of each of the plurality of types of food poisoning bacteria to a PCR solution, amplifying the amplification target region by PCR, and detecting the resulting amplified product.

10. The method for detecting food poisoning bacteria according to claim 9, wherein the culture medium comprises sodium chloride at a concentration of 1.5% w/v to 3.0% w/v.


SEQUENCE LISTING

<110>  TOYO SEIKAN KAISHA,LTD

<120>  Culture medium for culturing bacteria capable of causing food
poisoning and Detection method for bacteria capable of causing food
poisoning

<130>  TSK-1322-PCT

<160>  8

<170>  PatentIn version 3.1

<210>  1
<211>  31
<212>  DNA
<213>  Artificial

<220>
<223>  synthetic DNA

<400>  1
cgctcgtctg atgtccgcta ttccattgaa t                                    31


<210>  2
<211>  26
<212>  DNA
<213>  Artificial

<220>
<223>  synthetic DNA

<400>  2
accacgcagg caagctgctg agtcgg                                          26


<210>  3
<211>  30
<212>  DNA
<213>  Artificial

<220>
<223>  synthetic DNA

<400>  3
gaacaaccca tttgtattgg ttgttacggc                                      30


<210>  4
<211>  23
<212>  DNA
<213>  Artificial

<220>
<223>  synthetic DNA

<400>  4
ggctgctcgc ctttgctggt ttt                                             23


<210>  5

<211>  24
<212>  DNA
<213>  Artificial

<220>
<223>  synthetic DNA

<400>  5
cacgcctgga gagccttcac cagc                                              24


<210>  6
<211>  30
<212>  DNA
<213>  Artificial

<220>
<223>  synthetic DNA

<400>  6
gttactgtaa tggcgctggt catgtagctg                                        30


<210>  7
<211>  41
<212>  DNA
<213>  Artificial

<220>
<223>  synthetic DNA

<400>  7
tcagtttact tttttgggtt ttttggcttt catgaaacct g                           41


<210>  8
<211>  34
<212>  DNA
<213>  Artificial

<220>
<223>  synthetic DNA

<400>  8
tcattaatgt tcacagtcat gtaggatgtc agcc                                   34